# EUROPEAN PATENT APPLICATION

(11) **EP 3 015 074 A1**
(43) Date of publication of application: **04.05.2016**
(21) Application number: 15155728.7
(22) Date of filing: 19.02.2015
(51) Int. Cl.: A61B 8/08, A61B 8/00

(54) **Method and apparatus for displaying medical image**

(30) Priority: 29.10.2014 KR 20140148442
(71) Applicant: Samsung Medison Co., Ltd., Hongcheon-gun, Gangwon-do (KR)
(72) Inventor: Park, Jin-ki, Gangwon-do (KR); Song, Joo-hyun, Gangwon-do (KR)
(74) Representative: Land, Addick Adrianus Gosling

(57) **Abstract**

Provided are a method and apparatus for displaying a medical image. The method of displaying a medical image includes: acquiring volume data of an object; setting a cross-section of interest in the volume data; determining first and second sub-volume data within the volume data based on the cross-section of interest; generating medical images by respectively rendering the first and second sub-volume data in different directions,; and displaying the medical images.

## Description

This application claims the benefit of Korean Patent Application No. 10-2014-0148442, filed on October 29, 2014, in the Korean Intellectual Property Office, the disclosure of which is incorporated herein in its entirety by reference.

One or more exemplary embodiments relate to a method and apparatus for displaying a medical image, and more particularly, to a method and apparatus for displaying a three-dimensional (3D) medical image generated by rendering volume data of an object.

Due to its non-invasive and non-destructive nature, an ultrasound system has been widely used in many medical fields that require information about the inside of an object. The ultrasound system also plays a critical role in medical diagnosis since it can provide high-resolution images of internal tissues of an object to a medical practitioner without the need for performing a surgical procedure to directly incise the object for observation.

In general, an ultrasound system transmits ultrasound signals to an object and receives ultrasound signals (hereinafter, referred to as an 'echo signal') reflected from the object when a probe comes in contact with a surface of the object. The ultrasound system produces an ultrasound image of the object based on the echo signal received through the probe and displays the ultrasound image via a display.

One or more exemplary embodiments include a method and apparatus for displaying a medical image generated by rendering volume data in a plurality of directions based on a cross-section of interest of an object so as to observe a cross-section of the object from different angles.

Additional aspects will be set forth in part in the description which follows and, in part, will be apparent from the description, or may be learned by practice of the presented exemplary embodiments.

According to one or more exemplary embodiments, a method of displaying a medical image includes: acquiring volume data of an object; setting a cross-section of interest in the volume data; determining first and second sub-volume data within the volume data based on the cross-section of interest; generating medical images by respectively rendering the first and second sub-volume data in different directions,; and displaying the medical images.

The determining of the first and second sub-volume data may be performed by dividing the volume data based on the cross-section of interest.

The generating of the medical images may include: generating a first image by rendering the first sub-volume data in a first direction that is perpendicular to the cross-section of interest; and generating a second image by rendering the second sub-volume data in a second direction that is different from the first direction and is perpendicular to the cross-section of interest.

The method may further include: receiving information about a cross-section within the object from a user; and setting, based on the information about the cross-section, the cross-section of interest corresponding to the cross-section in the volume data.

The method may further include displaying a cross-section information image indicating which cross-section is the cross-section of interest in the object.

The method may further include receiving, from a user, display setting information necessary for setting regions of a screen for displaying the medical images.

In the displaying of the medical images, first and second images respectively generated by rendering the first and second sub-volume data in first and second directions may respectively be displayed in segmented regions of a screen.

The displaying of the medical images may include: displaying a first image generated by rendering the first sub-volume data in a first direction; and displaying a second image generated by rendering the second sub-volume data in a second direction so that the first and second images overlap each other.

In the displaying of the second image, the second image may have a smaller size than the first image and is displayed within the first image.

The acquiring of the volume data may include: transmitting an ultrasound signal to the object and acquiring ultrasound data based on an echo signal reflected from the object; and generating the volume data of the object based on the acquired ultrasound data.

According to one or more exemplary embodiments, an apparatus for displaying a medical image includes: a volume data acquisition unit configured to acquire volume data of an object; a processor configured to set a cross-section of interest in the volume data, determine first and second sub-volume data within the volume data based on the cross-section of interest, and generate medical images by respectively rendering the first and second sub-volume data in different directions; and a display configured to display the medical images.

According to one or more exemplary embodiments, a non-transitory computer-readable recording medium has recorded thereon a program for executing the method of displaying a medical image on a computer.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a block diagram of an apparatus for displaying a medical image according to an exemplary embodiment;
FIG. 2 is a diagram for explaining rendering performed by a processor according to an exemplary embodiment;
FIG. 3 is a diagram for explaining rendering performed by a processor according to another exemplary embodiment;
FIG. 4 is a block diagram of an apparatus for displaying a medical image according to another exemplary embodiment;
FIGS. 5 through 8 illustrate examples of screens displayed by a display according to an exemplary embodiment;
FIG. 9 is a diagram for explaining a method of displaying a medical image according to an exemplary embodiment;
FIG. 10 is a flowchart of a method of displaying a medical image, which is performed by an apparatus for displaying a medical image, according to an exemplary embodiment; and
FIG. 11 is a diagram for explaining an ultrasound apparatus including an apparatus for displaying a medical image, according to an exemplary embodiment.

### DETAILED DESCRIPTION

Reference will now be made in detail to exemplary embodiments, examples of which are illustrated in the accompanying drawings, wherein like reference numerals refer to like elements throughout. The exemplary embodiments set forth herein should be considered in a descriptive sense only and not for purposes of limitation. Other features or embodiments that will be readily apparent to those skilled in the art from the following descriptions and embodiments will be construed as being included in the present inventive concept.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies.

When a part "includes" or "comprises" an element, unless there is a particular description contrary thereto, the part can further include other elements, not excluding the other elements. In addition, terms such as "... unit", "... module", or the like refer to units that perform at least one function or operation, and the units may be implemented as hardware or software or as a combination of hardware and software. Expressions such as "at least one of," when preceding a list of elements, modify the entire list of elements and do not modify the individual elements of the list.

Throughout the specification, it will be understood that when an element is referred to as being "connected" or "coupled" to another element, it can be directly connected to or electrically coupled to the other element with one or more intervening elements interposed therebetween. When a part "includes" or "comprises" an element, unless there is a particular description contrary thereto, the part can further include other elements, not excluding the other elements.

Throughout the specification, an "object" may be a living or non-living thing of which image is to be generated. The object may also be a part of a human body. In this case, the object may include an internal organ such as the liver, the heart, the womb, the brain, a breast, or the abdomen, a fetus, etc. The object may also include a cross-section of the human body.

A "medical image" may be an ultrasound image or all other images generated using medical imaging techniques such as magnetic resonance imaging (MRI), computed tomography (CT), and positron emission tomography (PET). The images are obtained by reconstructing a cross-section or volume data of a tissue of a human body from a signal projected onto the tissue and are used for diagnosis and treatment of diseases.

Throughout the specification, a "user" may be, but is not limited to, a medical expert, for example, a medical doctor, a nurse, a medical laboratory technologist, a sonographer, or a medical imaging expert.

Exemplary embodiments will now be described in detail with reference to the accompanying drawings.

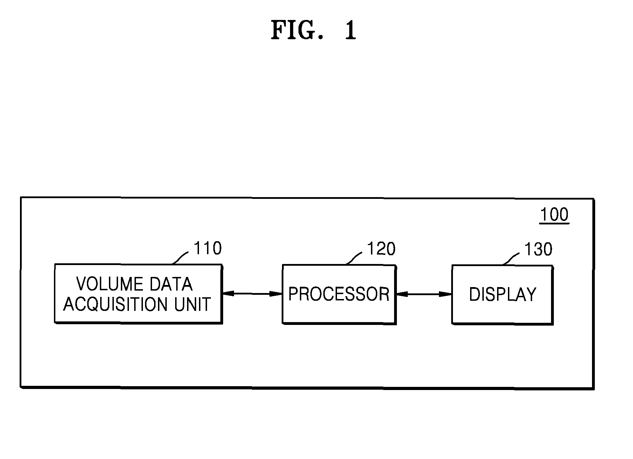

FIG. 1 is a block diagram of an apparatus 100 for displaying a medical image (hereinafter, referred to as a 'medical image displaying apparatus') according to an exemplary embodiment. The apparatus 100 may be a cart type apparatus or a portable type apparatus. The apparatus 100 refers to a device for providing a medical image of an object to a user via a screen. Examples of medical image display apparatus may include, but are not limited to, a picture archiving and communication system (PACS) viewer, a smartphone, a laptop computer, a personal digital assistant (PDA), and a tablet PC.

Referring to FIG. 1, the medical image display apparatus 100 may include a volume data acquisition unit 110, a processor 120, and a display 130. Although FIG. 1 shows that the medical image display apparatus 100 includes only components related to the present embodiment, it will be understood by those skilled in the art that the medical image display apparatus 100 may further include other components than those shown in FIG. 1

The volume data acquisition unit 110 may acquire volume data of an object. The volume data means data composed of a plurality of voxels. The volume data of the object may include spatial information of the object and clinical information such as anatomical shapes of tissue or organs of the object.

For example, the volume data acquisition unit 110 may create volume data of the object by using ultrasound data acquired from the object. As another example, the volume data acquisition unit 110 may acquire volume data from the outside via a communication module (not shown). As another example, the volume data acquisition unit 110 may acquire prestored volume data from a memory (not shown).

The processor 120 may be implemented as at least one of a central processing unit (CPU) and a graphics processing unit (GPU). According to an exemplary embodiment, the processor 120 performs rendering on volume data provided by the volume data acquisition unit 110 to generate a medical image. 'Rendering' is a technique used to generate and display a two-dimensional (2D) projection image of a three-dimensional (3D) discretely sampled data set such as volume data. According to an exemplary embodiment, the processor 120 may perform rendering by using ray casting projection.

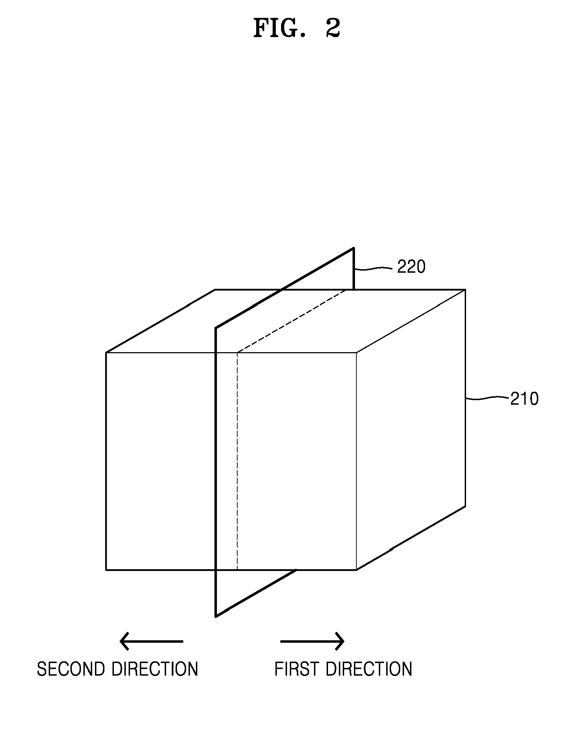

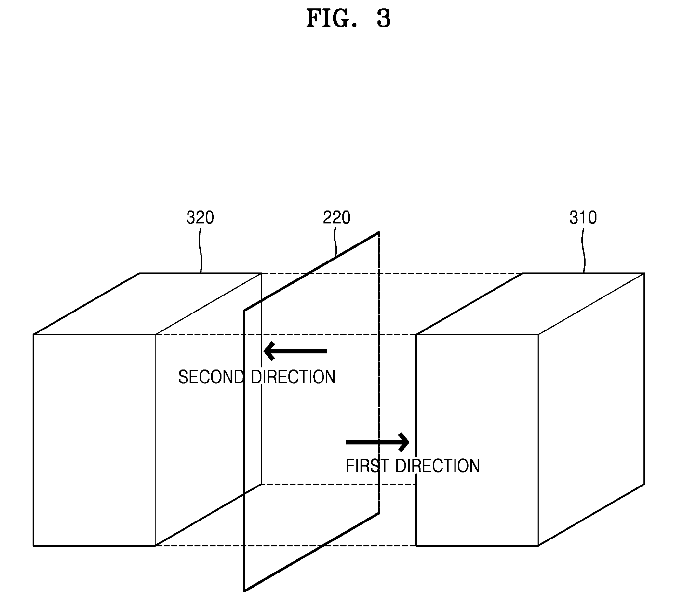

The processor 120 may also receive information about a cross-section of interest and set the cross-section of interest in volume data. The processor 120 may determine first and second sub-volume data in the volume data based on the set cross-section of interest. According to an exemplary embodiment, the processor 120 may determine the first and second sub-volume data by dividing the volume data based on the cross-section of interest. The processor 120 may render volume data in each of first and second directions that are perpendicular to the cross-section of interest. Furthermore, the processor 120 may render each of the first and second sub-volume data in a different direction to generate a medical image. A method of rendering volume data will now be described in more detail below with reference to FIGS. 2 and 3.

FIG. 2 is a diagram for explaining rendering performed by the processor 120 according to an exemplary embodiment.

Referring to FIGS. 1 and 2, the processor 120 may perform rendering on volume data 210. The processor 120 may set a cross-section of interest 220 in the volume data 210 and render the volume data 210 in each of first and second directions that are perpendicular to the cross-section of interest 220. According to an exemplary embodiment, the processor 120 may render the volume data 210 simultaneously in each of the first and second directions, thereby generating two images simultaneously.

FIG. 3 is a diagram for explaining rendering performed by the processor 120 according to another exemplary embodiment. Referring to FIGS. 1 through 3, the processor 120 may determine first and second sub-volume data 310 and 320 within the volume data 210 based on the cross-section of interest 220. According to an exemplary embodiment, the first and second sub-volume data 310 and 320 may be data obtained by dividing the volume data 210 along the cross-section of interest 220 Thus, the processor 120 may generate medical images by rendering the first and second sub-volume data 310 and 320 in two different directions, i.e., the first and second directions, respectively. According to an exemplary embodiment, the processor 120 may generate a first image by rendering the first sub-volume data 310 in the first direction that is perpendicular to the cross-section of interest 220. The processor 120 may also generate a second image by rendering the second sub-volume data 320 in the second direction that is different from the first direction and perpendicular to the cross-section of interest 220. Furthermore, according to an exemplary embodiment, the first and second directions may not be opposite directions as shown in FIGS. 2 and 3. For example, the first and second directions may form different angles or an angle for rendering the first or second sub-volume data 310 or 320 may be changed by a user input.

The display 130 of FIG. 1 may display information processed by the medical image display apparatus 100. For example, the display 130 may display an ultrasound image, or a user interface (UI) or graphic user interface (GUI) related to setting of functions of the medical image display apparatus 100.

The display 130 may include at least one selected from a liquid crystal display (LCD), a thin-film transistor-LCD (TFT-LCD), an organic light-emitting diode (OLED), a flexible display, a 3D display, and an electrophoretic display. According to exemplary embodiments, the medical image display apparatus 100 may include two or more displays 130.

The display 130 may display an image rendered by the processor 120 on a screen. In this case, the display 130 may display first and second images, which are generated by rendering volume data in first and second directions, respectively, on separate regions of the screen. Alternatively, the display 130 may display the first and second images to overlap on the screen.

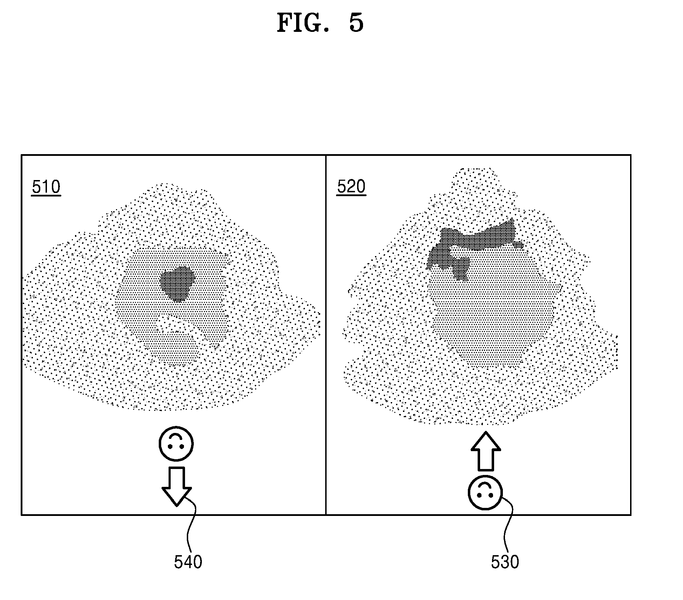

Furthermore, the display 130 may display a cross-section information image indicating which cross-section is a cross-section of interest in an object. Examples of screens displayed by the display 130 will be described in detail below with reference to FIGS. 5 through 8.

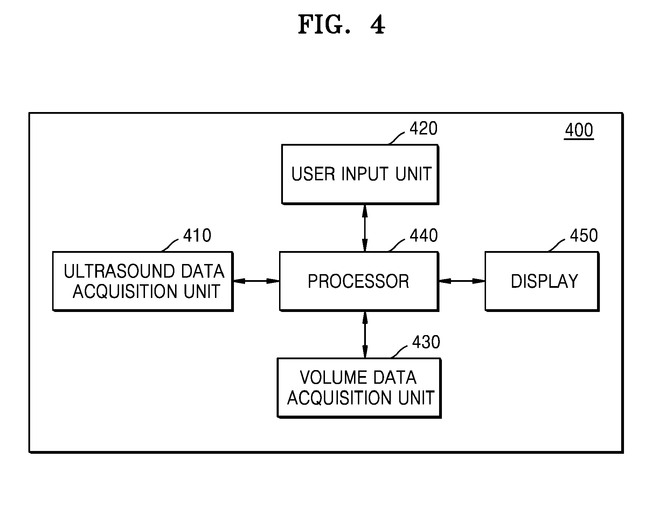

FIG. 4 is a block diagram of a medical image display apparatus 400 according to another exemplary embodiment.

Referring to FIG. 4, the medical image display apparatus 400 according to the present embodiment may include an ultrasound data acquisition unit 410, a volume data acquisition unit 430, a processor 440, a display 450, and a user input unit 420. Since the descriptions with respect to the apparatus of FIG. 1 may be applied to the volume data acquisition unit 430, the processor 440, and the display 450, the same descriptions as already presented with respect to FIG. 1 are omitted.

The ultrasound data acquisition unit 410 acquires ultrasound data related to an object. In detail, the ultrasound data acquisition unit 410 may transmit ultrasound signals to the object and acquire ultrasound data based on echo signals reflected from the object.

According to an exemplary embodiment, the ultrasound data acquisition unit 410 may include an ultrasound probe for transmitting or receiving ultrasound waves directly to or from an object. The ultrasound probe may transmit ultrasound signals to the object in response to a driving signal applied thereto and receive echo signals reflected from the object.

The ultrasound probe includes a plurality of transducers, and the plurality of transducers oscillate in response to electric signals and generate acoustic energy, that is, ultrasound waves. Furthermore, the ultrasound probe may be connected to a main body of the medical image display apparatus 400 by wire or wirelessly. According to exemplary embodiments, the medical image display apparatus 400 may include a plurality of ultrasound probes. An ultrasound probe according to an exemplary embodiment may include may include at least one selected from a 1-dimensional (1D) probe, a 1.5-dimenstional (1.5D) probe, a 2D (matrix) probe, and a 3D probe.

According to an exemplary embodiment, the volume data acquisition unit 430 may create volume data of an object by using ultrasound data provided by the ultrasound data acquisition unit 410. Furthermore, according to an exemplary embodiment, the volume data acquisition unit 430 may generate volume data by receiving ultrasound data from the outside via a communication module (not shown).

The user input unit 420 is a unit via which a user inputs data for controlling the medical image display apparatus 400. For example, the user input unit 420 may include a keypad, a dome switch, a touchpad (a capacitive overlay type, a resistive overlay type, an infrared beam type, a surface acoustic wave type, an integral strain gauge type, a piezoelectric type, etc.), a jog wheel, and a jog switch. However, exemplary embodiments are not limited thereto. Furthermore, the user input unit 420 may include a touch screen in which a display panel and a touch pad form a layered structure.

The user input unit 420 may receive information about a cross-section of an object from a user. The processor 440 may then set a cross-section of interest corresponding to a cross-section in the object in volume data based on the received information about a cross-section. According to an exemplary embodiment, the information about a cross-section may include information about a size and a position of the cross-section.

Furthermore, the user input unit 420 may receive display setting information required for setting a region of a screen on which medical images rendered by the processor 440 are to be displayed. Thus, the display 450 may partition the screen into a plurality of regions based on the display setting information received by the user input unit 420 and display the rendered medical images on the plurality of regions, respectively. The display 450 may also display the medical images to overlap in a single region of the screen. Examples of screens displayed by the display 450 will be described in greater detail below with reference to FIGS. 5 through 8.

Furthermore, the user input unit 420 may receive mode setting information necessary for setting a display mode of an image displayed on a screen from a user. According to an exemplary embodiment, the display mode may include an X-Ray mode, a Min mode, a Max mode, a Light mode, etc. In the X-Ray mode, a 3D ultrasound image may be formed using an average value of intensities of a plurality of voxels in volume data. The X-Ray mode is useful in displaying the 3D ultrasound image in a similar way to an X-Ray image. In the Max mode, a 3D ultrasound image is formed using a maximum intensity among intensities of voxels in volume data. The Max mode is useful in observing bones of a human body. In the Min mode, a 3D ultrasound image is formed using a minimum intensity among intensities of voxels in volume data. The Min mode is useful in observing vessels and hollows of a human body. In the Light mode, information about a depth of volume data is displayed as brightness.

FIG 5 illustrates an example of a screen showing medical images displayed by the display (450 of FIG. 4) according to an exemplary embodiment. In exemplary embodiments set forth below, an object is a blood vessel, but is not limited thereto.

The display 450 may partition the screen into first and second regions 510 and 520 and display rendered medical images on the first and second regions 510 and 520, respectively. In detail, the display 450 may partition the screen into the first and second regions 510 and 520 based on display setting information received from a user and display the rendered medical images on the first and second regions 510 and 520, respectively. A first image is obtained by rendering volume data in a first direction that is perpendicular to a cross-section of interest and displayed on the first region 510. A second image is obtained by rendering volume data in a second direction that is perpendicular to the cross-section of interest and displayed on the second region 520. Referring to FIG. 5, the first image showing a blood vessel observed in the first direction with respect to a predetermined cross-section of the blood vessel is displayed on the first region 510. The second image showing the blood vessel observed in the second direction that is opposite to the first direction is displayed on the second region 520. While FIG. 5 illustrates an example where the screen is divided into two regions having the same size, i.e., the first and second regions 510 and 520, exemplary embodiments are not limited thereto. The number, positions, or sizes of regions in the screen may vary according to display setting information.

A pair of smile icons & arrows 530 and 540 indicate that the first and second images are displayed on the first and second regions 510 and 520, respectively, show the blood vessel observed in different directions with respect to the predetermined cross-section thereof. The smile icon & arrow 530 indicates that the blood vessel is observed in the first direction with respect to the predetermined cross-section of the blood vessel. The smile icon & arrow 540 indicates that the blood vessel is observed in the second direction that is opposite to the first direction.

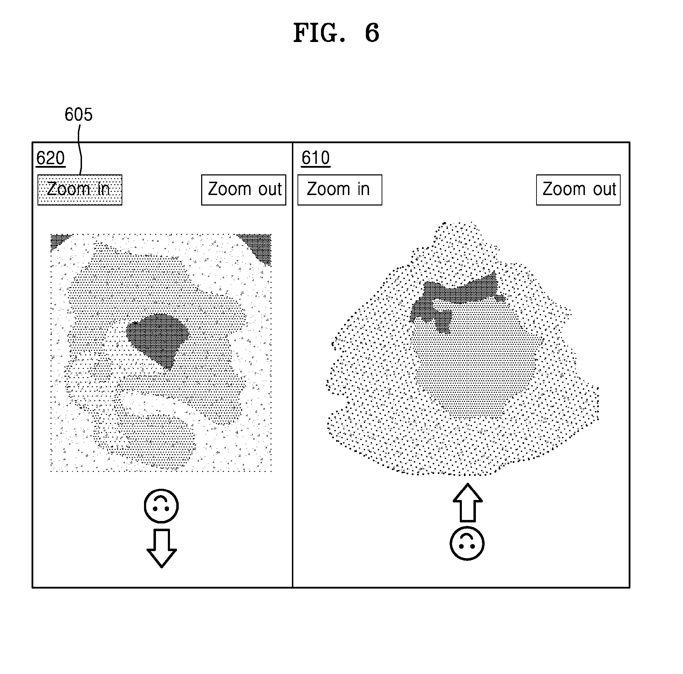

FIG. 6 illustrates another example of a screen displayed by the display (450 of FIG. 4) according to an exemplary embodiment.

The display 450 may reduce or enlarge a size of an image displayed on a screen based on a user input. The user input unit 420 may receive information about enlargement or reduction of an image displayed on a screen from a user. Referring to FIG. 6, the user input unit 420 may receive a user input (clicking a zoom in 605). The display 450 may then enlarge an image being displayed on a second region 620 based on the received user input and display the enlarged image. For example, the display 450 may enlarge the second image displayed on the second region (520 of FIG. 5) and display the enlarged image as an image on a second region 620.

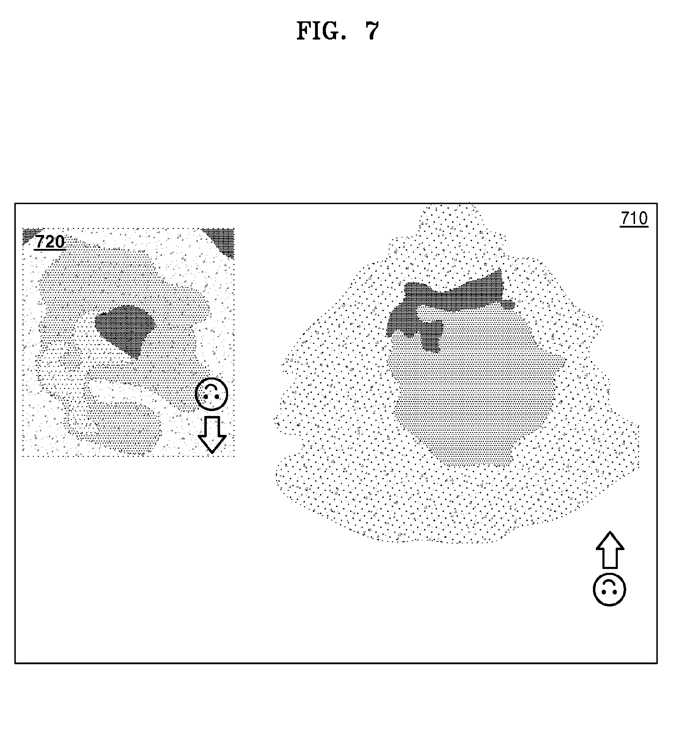

FIG. 7 illustrates another example of a screen displayed by the display (450 of FIG. 4) according to an exemplary embodiment.

The display 450 may respectively display rendered medical images in first and second regions 710 and 720 and overlap the first and second regions 710 and 720 on a single screen. Furthermore, based on display setting information received from a user, the display 450 may respectively display rendered medical images in the first and second regions 710 and 720 and overlap the first and second regions 710 and 720,. According to an exemplary embodiment, the display 450 may adjust a size of the second region 720 based on the display setting information and display the second region 720 at a position that is preset in the first region 710 by a default value or determined based on a user input. Furthermore, the display 450 may switch a medical image on the first region 710 with a medical image on the second region 720.

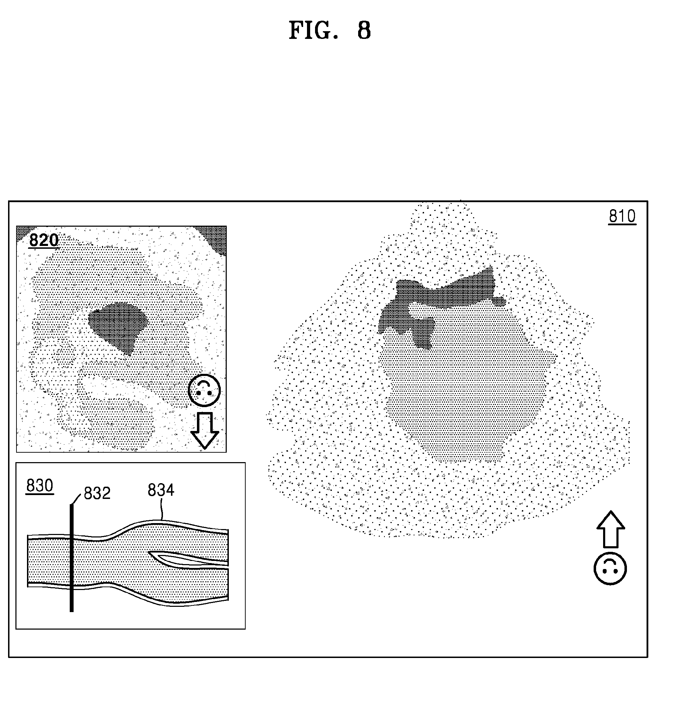

FIG. 8 illustrates another example of a screen displayed by the display (450 of FIG. 4) according to an exemplary embodiment.

The display 450 may display not only first and second regions 810 and 820 respectively corresponding to the first and second regions 710 and 720 of FIG. 7, but also a third region 830 showing a cross-section information image indicating which cross-sections in an object 834 are shown as the medical images in the first and second regions 710 and 720. In detail, as shown in FIG, 8, the display 450 may display the object 834 on the third region 830 to indicate that an image on the first region 810 is a medical image obtained by rendering volume data of the object 834 in a first direction that is perpendicular to a cross-section 832 in the object 834. Furthermore, the display 450 may display the object 834 on the third region 830 to indicate that an image on the second region 820 is a medical image obtained by rendering volume data of the object 834 in a second direction that is perpendicular to the cross-section 832 in the object 834.

In addition, a user may input information about the cross-section 832 in the object 834 to the user input unit 420 by referring to an image displayed on the third region 830. Thus, the processor 440 may set a cross-section of interest corresponding to the cross-section 832 on volume data so that medical images obtained by rendering volume data in first and second directions that are perpendicular to the cross-section of interest are displayed on the first and second regions 810 and 820, respectively. Although the display 450 displays the cross-section information image on the third region 830 as a 2D image, exemplary embodiments are not limited thereto, and the cross-section information image may be displayed in various forms, such as a 3D image, etc. according to user settings. Furthermore, the user may adjust the cross-section 832 via the user input unit (420 of FIG. 5) to change the cross-section of interest to another cross-section. In this case, the display 450 may display medical images generated based on the other cross-section on the first and second regions 810 and 820, respectively.

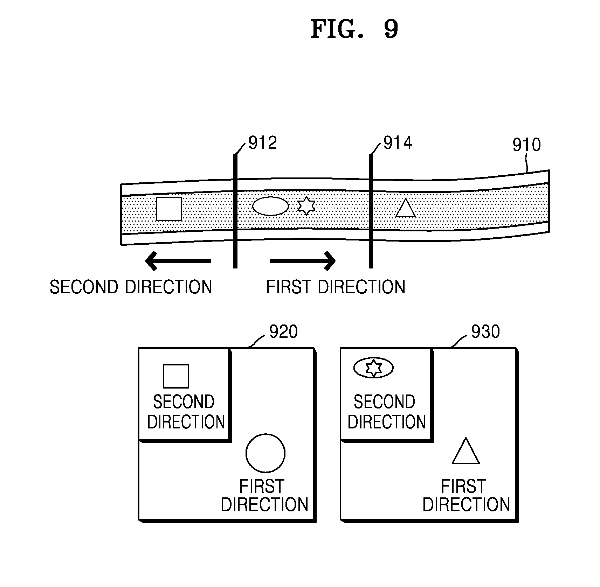

FIG. 9 is a diagram for explaining a method used by the display (450 of FIG. 4) to display a medical image according to an exemplary embodiment.

The display 450 may display an image corresponding to a cross-section 912 in an object 910 as an image 920. The display 450 may also display an image corresponding to a cross-section 914 as an image 930. In detail, the image 920 shows medical images obtained by rendering volume data of the object 910 in first and second directions based on the cross-section 912. Similarly, the image 930 shows medical images obtained by rendering volume data of the object 910 in first and second directions based on the cross-section 914.

Furthermore, the user may input information necessary for additionally setting a plurality of cross-sections in a direction from the cross-section 912 to the cross-section 914 in the object 910 to the user input unit (420 of FIG. 4). Each time a cross-section in the object 910 is set, the processor (440 of FIG. 4) may generate medical images by rendering the volume data of the object 910 in the first and second directions. In addition, the processor 440 may automatically set cross-sections in the object 910 and sequentially display medical images generated based on the set cross-sections, thereby producing an effect similar to that obtained when the inside of a body is seen as an endoscope is passed along a blood vessel.

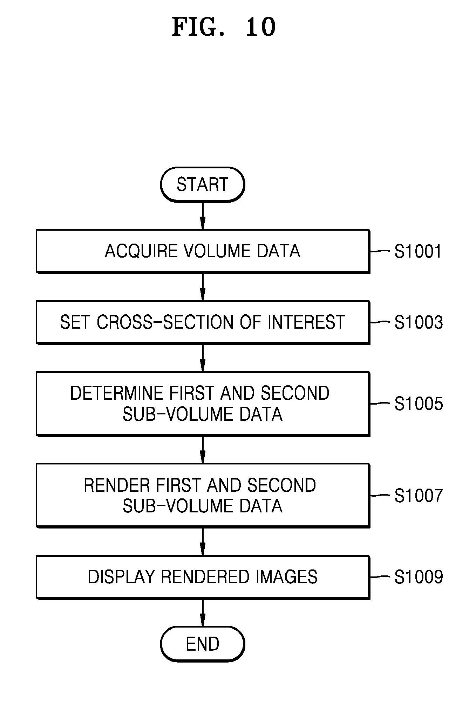

FIG. 10 is a flowchart of a method of displaying a medical image, which is performed by the medical image display apparatus (100 of FIG. 1 or 400 of FIG. 4), according to an exemplary embodiment.

Since the method of FIG. 10 may be performed by components in the medical image display apparatus (100 or 400), repeated descriptions with respect to FIGS. 1 and 4 are omitted.

The medical image display apparatus (100 or 400) may acquire volume data of an object (S1001). According to an exemplary embodiment, the medical image display apparatus (100 or 400) may acquire volume data of the object from the outside via a communication module. Alternatively, the medical image display apparatus (100 or 400) may acquire prestored volume data of the object from a memory. Furthermore, the medical image display apparatus (100 or 400) may create volume data of the object by acquiring ultrasound data.

The medical image display apparatus (100 or 400) may set a cross-section of interest on the volume data (S1003). The cross-section of interest may correspond to a cross-section in the object set by a user. In detail, the medical image display apparatus (100 or 400) may receive information about a cross-section in the object from the user and set a cross-section of interest corresponding to the received cross-section on the volume data based on the received information about the cross-section.

The medical image display apparatus (100 or 400) may determine first and second sub-volume data within the volume data based on the cross-section of interest (S1005). According to an exemplary embodiment, the medical image display apparatus (100 or 400) may determine the first and second sub-volume data by dividing the volume data based on the cross-section of interest.

The medical image display apparatus (100 or 400) may generate medical images by rendering the first and second sub-volume data in different directions, respectively (S1007). According to an exemplary embodiment, the medical image display apparatus (100 or 400) may render the volume data in first and second directions that are perpendicular to the cross-section of interest.

The medical image display apparatus (100 or 400) may display the medical images generated by rendering the first and second sub-volume data (S1009). According to an exemplary embodiment, the medical image display apparatus (100 or 400) may display a first image generated by rendering the first sub-volume data in the first direction and a second image generated by rendering the second sub-volume data in the second direction on separate regions of a screen, respectively. Alternatively, the medical image display apparatus (100 or 400) may display the medical images so as to overlap each other. The medical image display apparatus (100 or 400) may also display a cross-section information image indicating which cross-section in the object corresponds to the cross-section of interest set on the volume data. Furthermore, the medical image display apparatus (100 or 400) may receive display setting information necessary for setting regions of a screen on which medical images will be displayed from the user and display the medical images on the regions of the screen based on the received display setting information.

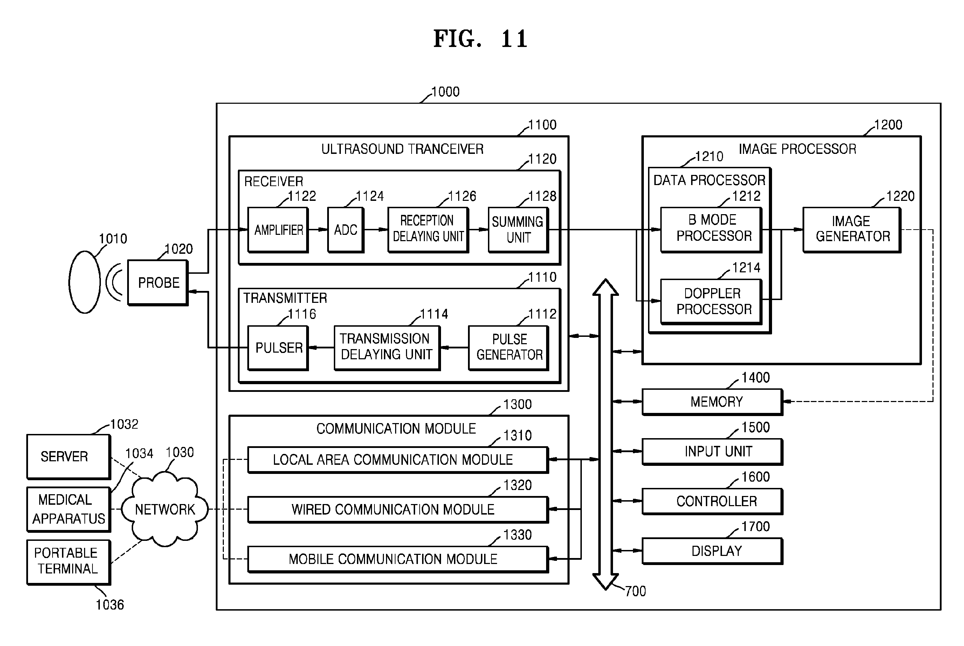

FIG. 11 is a diagram for explaining an ultrasound apparatus 1000 to which a medical image display apparatus can be applied, according to an exemplary embodiment.

A method of displaying a medical image according to an exemplary embodiment may be performed by the ultrasound apparatus 1000, and the medical image display apparatus (100 or 400) may be incorporated in the ultrasound apparatus 1000.

The medical image display apparatuses (100 and 400 of FIGS. 1 and 4) may perform some or all of the functions performed by the ultrasound apparatus 1000. The volume data acquisition unit 110 shown in FIG. 1 may correspond to a probe 1020, an ultrasound transceiver 1100, and an image processor 1200 shown in FIG. 11. The processor 120 shown in FIG. 1 may correspond to the image processor 1200 and a controller 1600 shown in FIG. 11. The display 130 may correspond to a display 1700 shown in FIG. 11. The ultrasound data acquisition unit 410 shown in FIG. 4 may correspond to the probe 1020 and the ultrasound transceiver 1100 shown in FIG. 11, and the user input unit 420 shown in FIG. 4 may correspond to an input unit 1500 shown in FIG. 11.

Referring to FIG. 11, the ultrasound apparatus 1000 may include a probe 1020, an ultrasound transceiver 1100, an image processor 1200, a communication module 1300, a display 1700, a memory 1400, an input unit 1500, and a controller 1600, which may be connected to one another via buses 700.

A transmitter 1110 supplies a driving signal to the probe 1020. The transmitter 1110 includes a pulse generator 1112, a transmission delaying unit 1114, and a pulser 1116. The pulse generator 1112 generates pulses for forming transmission ultrasound waves based on a predetermined pulse repetition frequency (PRF), and the transmission delaying unit 1114 delays the pulses by delay times necessary for determining transmission directionality. The pulses which have been delayed correspond to a plurality of piezoelectric vibrators included in the probe 1020, respectively. The pulser 1116 applies a driving signal (or a driving pulse) to the probe 1020 based on timing corresponding to each of the pulses which have been delayed.

A receiver 1120 generates ultrasound data by processing echo signals received from the probe 1020. The receiver 1120 may include an amplifier 1122, an analog-to-digital converter (ADC) 1124, a reception delaying unit 1126, and a summing unit 1128. The amplifier 1122 amplifies echo signals in each channel, and the ADC 1124 performs analog-to-digital conversion with respect to the amplified echo signals. The reception delaying unit 1126 delays digital echo signals output by the ADC 124 by delay times necessary for determining reception directionality, and the summing unit 1128 generates ultrasound data by summing the echo signals processed by the reception delaying unit 1126. Also, according to embodiments of the present invention, the receiver 1120 may not include the amplifier 1122. In other words, if the sensitivity of the probe 1020 or the capability of the ADC 1124 to process bits is enhanced, the amplifier 1122 may be omitted.

The image processor 1200 generates an ultrasound image by scan-converting ultrasound data generated by the ultrasound transceiver 1100 and displays the ultrasound image. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 1212 extracts B mode components from ultrasound data and processes the B mode components. An image generator 1220 may generate an ultrasound image indicating signal intensities as brightness based on the extracted B mode components.

Similarly, a Doppler processor 1214 may extract Doppler components from ultrasound data, and the image generator 1220 may generate a Doppler image indicating a movement of an object as colors or waveforms based on the extracted Doppler components.

According to an embodiment of the present invention, the image generator 1220 may generate a three-dimensional (3D) ultrasound image via volume-rendering with respect to volume data and may also generate an elasticity image by imaging deformation of the object 1010 due to pressure. Furthermore, the image generator 1220 may display various pieces of additional information in an ultrasound image by using text and graphics. In addition, the generated ultrasound image may be stored in the memory 1400.

The communication module 1300 is connected to a network 1030 by wire or wirelessly to communicate with an external device or a server. The communication module 1300 may exchange data with a hospital server or another medical apparatus in a hospital, which is connected thereto via a PACS. Furthermore, the communication module 1300 may perform data communication according to the digital imaging and communications in medicine (DICOM) standard.

The communication module 1300 may transmit or receive data related to diagnosis of an object, e.g., an ultrasound image, ultrasound data, and Doppler data of the object, via the network 1030 and may also transmit or receive medical images captured by another medical apparatus, e.g., a computed tomography (CT) apparatus, a magnetic resonance imaging (MRI) apparatus, or an X-ray apparatus. Furthermore, the communication module 1300 may receive information about a diagnosis history or medical treatment schedule of a patient from a server and utilizes the received information to diagnose the patient. Furthermore, the communication module 1300 may perform data communication not only with a server or a medical apparatus in a hospital, but also with a portable terminal of a medical doctor or patient.

The communication module 1300 is connected to the network 1030 by wire or wirelessly to exchange data with a server 1032, a medical apparatus 1034, or a portable terminal 1036. The communication module 1300 may include one or more components for communication with external devices. For example, the communication module 1300 may include a local area communication module 1310, a wired communication module 1320, and a mobile communication module 1330.

The local area communication module 1310 refers to a module for local area communication within a predetermined distance. Examples of local area communication techniques according to an embodiment of the present invention may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 1320 refers to a module for communication using electric signals or optical signals. Examples of wired communication techniques according to an embodiment of the present invention may include communication via a twisted pair cable, a coaxial cable, an optical fiber cable, and an Ethernet cable.

The mobile communication module 1330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 1400 stores various data processed by ultrasound apparatus 1000. For example, the memory 1400 may store medical data related to diagnosis of an object, such as ultrasound data and an ultrasound image that are input or output, and may also store algorithms or programs which are to be executed in ultrasound apparatus 1000.

The memory 1400 may be any of various storage media, e.g., a flash memory, a hard disk drive, EEPROM, etc. Furthermore, ultrasound apparatus 1000 may utilize web storage or a cloud server that performs the storage function of the memory 1400 online.

The input unit 1500 refers to a means via which a user inputs data for controlling ultrasound apparatus 1000. The input unit 1500 may include hardware components, such as a keypad, a mouse, a touch panel, a touch screen, and a jog switch. However, embodiments of the present invention are not limited thereto, and the input device 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

A display 1700 displays the generated ultrasound image. The display 1700 may display not only an ultrasound image, but also various pieces of information processed by the ultrasound apparatus 1000 on a screen image via a graphical user interface (GUI). In addition, ultrasound apparatus 1000 may include two or more displays 1700 according to embodiments of the present invention.

The controller 1600 may control all operations of ultrasound apparatus 1000. In other words, the controller 1600 may control operations among the probe 1020, the ultrasound transceiver 1100, the image processor 1200, the communication module 1300, the memory 1400, and input unit 1500 shown in FIG. 11.

All or some of the probe 1020, the ultrasound transceiver 1100, the image processor 1200, the communication module 1300, the memory 1400, input unit 1500, and the controller 1600 may be implemented as software modules. However, embodiments of the present invention are not limited thereto, and some of the components stated above may be implemented as hardware modules. Furthermore, at least one selected from the ultrasound transceiver 1100, the image processor 1200, and the communication module 1300 may be included in the controller 1600. However, embodiments of the present invention are not limited thereto.

Examples of the above-described system include a processor, a memory for storing and executing program data, a permanent storage such as a disc drive, a communication port for communicating with external devices, and a user interface device such as a touch panel, keys, or buttons. Methods implemented by using software modules or algorithms may be stored as computer-readable codes executable by the processor or program instructions on a computer-readable recording medium. Examples of the computer-readable recording medium include magnetic storage media (e.g., ROM, RAM, floppy disks, hard disks, etc.) and optical recording media (e.g., CD-ROMs and Digital Versatile Discs (DVDs)). The computer-readable recording medium may also be distributed over network-coupled computer systems so that the computer-readable codes are stored and executed in a distributed fashion. The computer-readable recording medium can be read by a computer, function as a memory, and executed by the processor.

Exemplary embodiments may be described in terms of functional block components and various processing steps. Such functional blocks may be realized by any number of hardware and/or software components configured to perform the specified functions. For example, the exemplary embodiments may employ various integrated circuit components, e.g., memory elements, processing elements, logic elements, look-up tables, and the like, which may carry out a variety of functions under the control of one or more microprocessors or other control devices. Similarly to where the elements of the exemplary embodiments are implemented using software programming or software elements, the exemplary embodiments may be implemented with any programming or scripting language such as C, C++, Java, assembler, or the like, with the various algorithms being implemented with any combination of data structures, processes, routines or other programming elements. Functional aspects may be implemented in algorithms that are executed on one or more processors. Furthermore, the exemplary embodiments may employ any number of conventional techniques for electronics configuration, signal processing and/or data processing and the like. The words "mechanism", "element", "means", and "construction" are used broadly and are not limited to mechanical or physical configurations, but may include software routines in conjunction with processors, etc.

The particular implementations shown and described herein are illustrative examples and are not intended to otherwise limit the scope of the inventive concept in any way. For the sake of brevity, conventional electronics, control systems, software development and other functional aspects of the systems may not be described in detail. Furthermore, the connecting lines or connectors shown in the various figures presented are intended to represent exemplary functional relationships and/or physical or logical couplings between the various elements.

The use of the term "the" and similar referents in the context of describing the exemplary embodiments (especially in the context of the following claims) are to be construed to cover both the singular and the plural. Furthermore, recitation of ranges of values herein are merely intended to serve as a shorthand method of referring individually to each separate value falling within the range, unless otherwise indicated herein, and each separate value is incorporated into the specification as if it were individually recited herein. Finally, the operations of all methods described herein can be performed in any suitable order unless otherwise specified herein or otherwise clearly contradicted by context. The use of any and all examples, or exemplary language (e.g., "such as") provided herein, is intended merely to better illuminate the invention and does not pose a limitation on the scope of the inventive concept unless otherwise claimed. While the present general inventive concept has been particularly shown and described with reference to exemplary embodiments thereof, it will be understood by those of ordinary skill in the art that various changes, combinations, and modifications in form and details may be made therein according to design conditions and factors without departing from the spirit and scope of the present inventive concept as defined by the following claims and their equivalents.

## Claims

1. A method of displaying a medical image, the method comprising:
acquiring volume data of an object;
setting a cross-section of interest in the volume data;
determining first and second sub-volume data within the volume data based on the cross-section of interest;
generating medical images by respectively rendering the first and second sub-volume data in different directions; and
displaying the medical images.

2. The method of claim 1, wherein the determining of the first and second sub-volume data is performed by dividing the volume data based on the cross-section of interest.

3. The method of claim 1 or 2, wherein the generating of the medical images comprises:
generating a first image by rendering the first sub-volume data in a first direction that is perpendicular to the cross-section of interest; and
generating a second image by rendering the second sub-volume data in a second direction that is different from the first direction and is perpendicular to the cross-section of interest.

4. The method of claim 1, 2 or 3, further comprising:
receiving information about a cross-section within the object from a user; and
setting, based on the information about the cross-section, the cross-section of interest corresponding to the cross-section in the volume data.

5. The method of any of claims 1-4, further comprising displaying a cross-section information image indicating which cross-section is the cross-section of interest in the object.

6. The method of any of claims 1-5, further comprising receiving, from a user, display setting information necessary for setting regions of a screen for displaying the medical images.

7. The method of any of claims 1-6, wherein in the displaying of the medical images, first and second images respectively generated by rendering the first and second sub-volume data in first and second directions are respectively displayed in segmented regions of a screen.

8. The method of any of claims 1-7, wherein the displaying of the medical images comprises:
displaying a first image generated by rendering the first sub-volume data in a first direction; and
displaying a second image generated by rendering the second sub-volume data in a second direction so that the first and second images overlap each other.

9. The method of claim 8, wherein in the displaying of the second image, the second image has a smaller size than the first image and is displayed within the first image.

10. The method of any of claims 1-9, wherein the acquiring of the volume data comprises:
transmitting an ultrasound signal to the object and acquiring ultrasound data based on an echo signal reflected from the object; and
generating the volume data of the object based on the acquired ultrasound data.

11. An apparatus for displaying a medical image, the apparatus comprising:
a volume data acquisition unit configured to acquire volume data of an object;
a processor configured to set a cross-section of interest in the volume data, determine first and second sub-volume data within the volume data based on the cross-section of interest, and generate medical images by respectively rendering the first and second sub-volume data in different directions; and
a display configured to display the medical images.

12. The apparatus of claim 11, wherein the processor generates a first image by rendering the first sub-volume data in a first direction that is perpendicular to the cross-section of interest and generates a second image by rendering the second sub-volume data in a second direction that is different from the first direction and is perpendicular to the cross-section of interest.

13. The apparatus of claim 11 or 12, further comprising a user input unit configured to receive information about a cross-section within the object from a user,
wherein the processor sets the cross-section of interest corresponding to the cross-section in the volume data based on the information about the cross-section.

14. The apparatus of claim 11, 12 or 13, wherein the display respectively displays first and second images generated by respectively rendering the first and second sub-volume data in first and second directions in segmented regions of a screen.

15. A non-transitory computer-readable recording medium having recorded thereon a program for executing the method of any one of claims 1 through 10 on a computer, and/or a apparatus wherein the method according to any one claims 1-10 is used.
